# EUROPÄISCHE PATENTSCHRIFT

(11) **EP 2 707 084 B1**
(45) Veröffentlichungstag und Bekanntmachung des Hinweises auf die Patenterteilung: **02.03.2022**
(21) Anmeldenummer: 12720804.9
(22) Anmeldetag: 04.05.2012
(51) Int. Cl.: A61N 1/04

(54) **ELEKTRODE MIT EINER EINGEBETTETEN SCHICHT UND EIN VERFAHREN ZU DEREN HERSTELLUNG**
ELECTRODE WITH AN EMBEDDED LAYER AND A METHOD FOR ITS MANUFACTURE
ÉLECTRODE AVEC UNE COUCHE NOYÉE ET UN PROCÉDÉ POUR SA FABRICATION

(30) Priorität: 12.05.2011 DE 102011101583
(43) Veröffentlichungstag der Anmeldung: 19.03.2014
(73) Patentinhaber: Ottobock SE & Co. KGaA, 37115 Duderstadt (DE)
(72) Erfinder: ANHALT, Klaus-Peter, 37434 Rhumspringe (DE); REINHARDT, Holger, 47906 Kempen (DE); OPITZ, Lucien, 31832 Springe (DE)
(74) Vertreter: Friedrich, Andreas
(86) Internationale Anmeldenummer: PCT/EP2012/001925
(87) Internationale Veröffentlichungsnummer: WO 2012/152418

(56) Entgegenhaltungen:
- DE-A1- 2 830 219
- DE-A1-102005 058 850
- GB-A- 2 432 323
- US-A1- 2006 052 683

## Beschreibung

Die Erfindung betrifft eine Elektrode zum transkutanen Übertragen elektrischer Signale mit einer ersten Schicht und einer elektrisch leitfähigen Polymerschicht, die leitfähige Partikel enthält, wobei die erste Schicht teilweise von der Polymerschicht durchdrungen ist, so dass sie aus der Polymerschicht an zumindest einer ersten Seite herausragt, sowie ein Verfahren zum Herstellen einer solchen Elektrode sowie eine Bandage mit mindestens einer derartigen Elektrode.

Derartige Elektroden werden für unterschiedliche klinische Anwendungen, beispielsweise Elektrokardiogramme, Elektroenzephalogramme oder Elektromyogramme verwendet, finden jedoch auch bei längerfristigen Verfahren Anwendung. So werden derartige Elektroden insbesondere auch für die Muskelstimulation, beispielsweise zum Muskelaufbau durch funktionelle Elektrostimulation (FES) oder transkutane elektrische Nervenstimulation (TENS), eingesetzt.

Dabei werden elektrische Signale über die Elektroden auf den Körper des Trägers der Elektroden übertragen, so dass die Muskeln zur Bewegung angeregt werden. Alternativ dazu ist es auch möglich, mit derartigen Elektroden elektrische Signale von einem Muskel bzw. der Haut des Trägers abzunehmen und der weiteren Verarbeitung zuzuführen.

Um die Übertragung der elektrischen Signale von der Elektrode auf den Körper des Patienten möglich zu machen, ist es nötig, dass der tatsächliche Kontakt zwischen der Elektrode und der Haut des Patienten über ein ionisch leitendes Medium erfolgt. Lediglich die Ableitung elektrischer Signale von der Haut des Patienten ist beispielsweise auch durch metallische Elektroden möglich. So ist es beispielsweise aus der DE 10 2009 013 470 A1 bekannt, ein Gelkissen mit einem ionisch leitfähigen Liquidgel auf der der Haut zugewandten Seite der Elektrode anzuordnen. Auf der anderen Seite des Gelkissens befindet sich dann ein flüssigkeitsdichtes Trägermaterial, um ein Austreten des Liquidgels auf dieser Seite der Elektrode zu verhindern. Um einen möglichst guten elektrischen Kontakt zwischen der Elektrode, insbesondere dem Liquidgel im Gelkissen und der Haut des Trägers zu gewährleisten, muss auf der Haut des Patienten ein Gel- be-ziehungsweise Feuchtigkeitsfilm gebildet werden. Die mit diesem Hydro- oder Liquidgel versehenen Elektroden sind sehr klebrig, so dass sie für eine zumindest kurzfristig gute Haftung an der Haut des Trägers sorgen. Schwammelektroden hingegen werden durch die einfach herausdrückbare Flüssigkeit feucht und schmierig.

Die Kontaktierung des ionisch leitenden Gelkissens findet über eine Metallplatte oder eine Gummielektrode statt. Diese weist an der dem Gelkissen abgewandten Seite einen Metalldruckknopf auf, mit dem sie beispielsweise von einem EKG-Gerät über dessen Kabel kontaktiert werden kann.

Die Metallplatte muss dabei mit dem Gelkissen in ständigem Berührungs-kontakt stehen, um eine elektrische Kontaktierung zu gewährleisten.

Ein ähnlicher Elektrodenaufbau ist beispielsweise aus der EP 1 021 986 A2 bekannt. Auch hier befindet sich an einer der Haut zugewandten Seite eines Trägermaterials ein Gelkörper, der mit einem Leitgel gefüllt ist. An dem Gelkissen ist ein metallischer Sensor mit einem Druckknopfanschlusselement angeordnet, über den der Sensor und damit das Gelkissen kontaktierbar ist. Auch hier muss der metallische Sensor in ständigem Berührungskontakt zum Gelkissen stehen, um eine elektrische Kontaktierung zu gewährleisten.

Aus der EP 0 467 966 B1 ist eine elektrische Reizelektrode bekannt, bei der die elektrischen Signale über ein leitfähiges Tuch direkt in die Haut des Patienten eingekoppelt werden. Dieses leitfähige Tuch weist eine leitfähige Fasereinrichtung auf, und ist mit einem leitfähigen Klebemittel getränkt, das hier für den eigentlichen Kontakt zwischen der Elektrode und der Haut sorgt.

Alle bisher genannten Elektroden sind für längere Tragedauer, beispiels-weise bei längeren Behandlungen in Form von Muskelstimulationen, die sich oftmals über mehrere Tage erstrecken, ungeeignet. Im Laufe dieser Zeit kommt es beispielsweise aufgrund der Muskelkontraktionen, die durch die elektrischen Signale hervorgerufen werden, die durch die Elektroden in den menschlichen Körper eingebracht werden, zu einer starken Bewegung der Haut am Ort der Elektrode. Zudem bewegen sich die Patienten im Laufe mehrerer Tage stark, so dass an die Elektroden hohe Anforderungen in Bezug auf mechanische Festigkeit gestellt werden.

Nachteilig ist zum einen, dass die bisher beschriebenen Gelkissen auf-grund des darin enthaltenen Liquidgels sehr klebrig sind. Kommt es nun aufgrund von Bewegungen des Patienten zu einer Relativbewegung zwischen der Haut und der an ihr klebenden Elektrode, kann sich die Klebe-stelle lösen und bei der nächsten Bewegung an einer anderen Stelle wie-der bilden. Dies hat einen ähnlichen Effekt, wie wenn ein Pflaster ständig gelöst und wieder angeklebt wird. Es führt zu Hautreizungen und Irritationen und ist allgemein für den Träger unangenehm. Zudem hat ein derartiges Ablösen und Wiederankleben der Elektrode den Nachteil, dass zumindest kurzfristig der elektrische Kontakt zwischen Elektrode und dem Kör-per des Patienten unterbrochen oder zumindest beeinträchtigt wird. Zum anderen kann es aufgrund der Relativbewegung zwischen der Haut des Patienten und der Elektrode zu einer Verschiebung kommen, so dass die optimale Positionierung der Elektrode relativ zu dem von ihr stimulierten Muskel nicht mehr gewährleistet werden oder eine Deformation der Elektrode bewirkt werden kann.

Neben dem selbstklebenden Gelkissen weist auch die Kontaktierung über einen metallischen Sensor Nachteile auf. Aufgrund der starken Bewegung, der eine Elektrode im Laufe der Zeit am menschlichen Körper ausgesetzt ist, kann es hier zu Brüchen in der Kontaktierung kommen, so dass im schlimmsten Fall der elektrische Kontakt unterbrochen wird, so dass keine weiteren Stromsignale in den Körper des Patienten eingebracht werden können. Dies hätte ein Scheitern der Therapie zur Folge.

Aus der WO 01/02052 A2 ist ein Kleidungsstück bekannt, das wenigstens zwei unterschiedliche Zonen umfasst, von denen eine elektrisch leitfähig ausgebildet ist. Beide Gewebearten werden miteinander vernäht beziehungsweise verstrickt. Die elektrisch leitfähigen Zonen können dann als Elektrode für medizinische Anwendungen verwendet werden.

Die US 5,123,423 offenbart ein Pad für Defibrillatoren, das zwischen die Elektrode des Defibrillators und die Haut des Patienten geklebt wird. Bei diesen Pads kommt es aufgrund der Notfallsituation, in der sie verwendet werden, auf erhöhten Tragekomfort nicht an. Die Pads bestehen aus einer unteren Schicht, die aus einem leitfähigen Polymer besteht. Darin und darüber ist eine faserige Schicht beispielsweise aus Kohlefasern angeordnet, die verhindert, dass die Polymerschicht auch an der Elektrode des Defibrillators klebt.

Aus der US 2007/0049814 A1 ist ein Set aus Kleidungsstücken bekannt, das über Elektroden und Gelreservoirs verfügt, durch die elektrische Signale als Stimulation an unterhalb des Kleidungsstückes liegende Muskeln des Trägers gesendet werden können.

Die DE 93 16 259 U1 offenbart eine Elektrode, die aus einer leitenden Polymerschicht und einer diese Schicht abdeckenden Abdeckschicht besteht. Dabei wird die Polymerschicht in eine Ausnehmung einer Schaumstoff-schicht eingegossen, um eine bündige Verbindung der leitenden und nicht leitenden Stoffschichten zu erreichen.

Aus der DE 10 2009 017 179 A1 ist eine Vorrichtung zur Elektromyostimulation bekannt, die aus einem ein- oder mehrteiligen Anzug besteht, in den integrierte textilbasierte Elektroden, Feuchtigkeitsspender und elektrische Zuleitungen eingefügt sind.

Der Erfindung liegt somit die Aufgabe zugrunde, eine Elektrode und ein Verfahren zum Herstellen einer solchen Elektrode vorzuschlagen, die auch bei starken Bewegungen und langer Tragedauer ein für den Patienten an-genehmes Traggefühl bei gleichzeitig optimaler Kontaktierung gewährleistet.

Die Erfindung löst die gestellte Aufgabe durch eine gattungsgemäße Elektrode zum transkutanen Übertragen elektrischer Signale mit einer ersten Schicht, die ausgebildet ist, eine Flüssigkeit zumindest auch aufgrund einer Kapillarkraft festzuhalten, und einer elektrisch leitfähigen Polymerschicht, die leitfähige Partikel enthält, wobei die erste Schicht teilweise von der Polymerschicht durchdrungen ist, so dass sie aus der Polymerschicht an zumindest einer ersten Seite herausragt. Zudem umfasst die Elektrode erfindungsgemäß zusätzlich eine Elektrodenkontaktierungsschicht, die zumindest teilweise von der Polymerschicht durchdrungen ist, so dass sie auf einer der ersten Seite gegenüberliegenden Seite aus der Polymerschicht herausragt. Die erste Schicht ist ein Schwamm oder ein Faservlies. An dieser Elektrodenkontaktierungsschicht ist vorteilhafterweise ein Anschlussdraht oder ein sonstiger elektrischer Kontakt angeordnet, um von der Elektrode aufgenommene elektrische Signale der Weiterverarbeitung zuzuführen oder elektrische Signale zur Elektrode zu leiten, die von der Elektrode auf den Patienten übertragen werden sollen.

Vorteilhafterweise handelt es sich bei der ersten Schicht um ein Faservlies, insbesondere ein Nanofaservlies. Da die erste Schicht ausgebildet ist, eine Flüssigkeit zumindest auch aufgrund einer Kapillarkraft festzuhalten, werden die Nachteile, die durch die Verwendung eines Hydrogelkissens hervorgerufen werden, wirksam verhindert. Als erste Schicht ist erfindungsgemäß ein Schwamm oder ein Faservlies, insbesondere ein Mikro- oder Nanofaservlies verwendet werden. Je kleiner die Öffnungen und Kavitäten in der ersten Schicht sind, desto größer ist die daraus entstehende Kapillarkraft, wodurch die Fähigkeit der ersten Schicht, die Flüssigkeit festzuhalten, steigt. Insbesondere bei der Verwendung von Mikro- und Nanofaservliesen ist die Kapillarkraft hoch. Es ist oftmals völlig ausreichend, die erste Schicht beispielsweise nur leicht zu befeuchten, da durch die sehr hohe Kapillarkraft insbesondere eines Nanofaservlieses diese Feuchtigkeit in der ersten Schicht sehr stark gebunden ist. Es ist sogar denkbar, die Elektroden so auszugestalten, dass sie im trockenen Zustand verwendet werden können und lediglich durch auf der Haut des Patienten vorhandenen Schweiß ausreichend Feuchtigkeit in die erste Schicht der Elektrode eindringen kann, um für eine elektrisch leitfähige Verbindung zu sorgen.

Als besonders bevorzugte Ausgestaltung hat sich, wie bereits dargelegt, das Nanofaservlies als erste Schicht erwiesen. Im Folgenden wird die Erfindung anhand eines Ausführungsbeispiels mit einem Nanofaservlies beschrieben. Die gesamten das Nanofaservlies betreffenden Ausführungen sind jedoch ebenso für erste Schichten zutreffend, die aus anderen Materialien hergestellt wurden. Wenn im Folgenden folglich von einem Nanofaservlies die Rede ist, ist immer auch eine erste Schicht gemeint, die in einer anderen Ausgestaltung vorliegen kann.

Das Nanofaservlies, das insbesondere im Elektrospinverfahren hergestellt werden kann, bildet dabei die Kontaktfläche zur Haut des Patienten. Wird das Vlies beispielsweise im Elektrospinverfahren hergestellt, entstehen sehr dünne Fasern, beispielsweise aus einer Polymerlösung durch die Behandlung in einem elektrischen Feld. Es entstehen typischerweise Fasern mit einem Durchmesser kleiner als 1000 nm, weswegen hier von Nanofasern die Rede ist. Ein derartiges Vlies weist eine Vielzahl kleiner und kleinster Zwischenräume auf. In diesen ist die Kapillarwirkung aufgrund des sehr geringen Durchmessers so stark, dass eine darin enthaltene Flüssigkeit mechanisch und physikalisch gebunden ist und in dem Vlies gespeichert wird. Ist das Nanofaservlies nun mit einer Flüssigkeit getränkt, die Ionen enthält, ist das Vlies dadurch ionisch leitfähig, selbst wenn das eigentliche Vliesmaterial elektrisch isolierend sein sollte. Wird also eine elektrische Spannung angelegt, fließen die Ionen zu dem entsprechenden Punkt, so dass es zu einem Stromfluss kommt.

Dadurch, dass das Wasser bzw. die mit Ionen versetzte Flüssigkeit aufgrund der starken Kapillarwirkung im Nanofaservlies physikalisch stärker als bei anderen verwendeten Materialien, beispielsweise einem Schwamm, gebunden ist und bei der Verdunstung deutlich behindert wird, kann auf hygroskopische Zusätze und Additive verzichtet werden, so dass dieses Nanofaservlies nicht klebrig ist. Die aus dem Stand der Technik bekannten Nachteile der Hydrogel bzw. Liquidgelkissen, die ein ständiges Ablösen und Ankleben der Elektrode zur Folge hatten, sind somit wirksam vermieden worden. Die Kombination aus einem Faservlies und einem ionisch leitfähigen Medium gewährleistet eine optimale Kontaktierung der Haut des Trägers der Elektrode. Dadurch, dass die Elektroden gemäß dem Ausführungsbeispiel der vorliegenden Erfindung nicht an der Haut kleben, wird es insbesondere an Körperpositionen, die einer starken Bewegung unterworfen sein können, beispielsweise Knie oder Ellenbogen, möglich, auch bei starker Bewegung dieser Körperregionen eine optimale Kontaktierung zu erreichen, ohne dass es zu Hautspannungen oder teilweisen Ablösungen der Elektrode kommt.

Das Nanofaservlies ist teilweise von einer Polymerschicht durchdrungen, die leitfähige Partikel enthält. Dabei handelt es sich jedoch nicht um ionisch leitfähige Partikel, sondern um elektronisch leitfähige Partikel, in denen der Stromfluss folglich durch eine Bewegung der Elektronen zustande kommt. Dadurch, dass das Nanofaservlies teilweise von der Polymerschicht druchdrungen ist, dringt das Polymer mit den darin enthaltenen leitfähigen Partikeln in diesem Bereich in die kleinen und kleinsten Zwischenräume des Nanofaservlieses ein. Da das Nanofaservlies jedoch nur teilweise von der Polymerschicht durchdrungen ist, wird auch nur ein Teil der Zwischenräume des Nanofaservlieses von dem Polymer durchdrungen und aufgefüllt. Die übrigen bleiben mit der Ionen beinhaltenden Flüssigkeit befüllbar. Auf diese Weise kommt es zu einer optimalen Kontaktierung zwischen den ionisch leitenden Flüssigkeit im Nanofaservlies und den elektronisch leitenden Partikeln in der Polymerschicht. Vorzugsweise ist das Nanofaservlies etwa zu einem Drittel bis zur Hälfte vom Polymer umgeben, während der Rest des Nanofaservlieses auf der der Haut zugewandten Seite der Elektrode aus der Polymerschicht hervorsteht. Bei einer Dicke des Nanofaservlieses von beispielsweise 370 m beträgt die Eindringtiefe beispielsweise 130 m.

Sowohl ein Nanofaservlies, beispielsweise ein Elektrospinvlies, als auch eine Polymerschicht sind biegsam und flexibel ausgebildet, so dass der gesamte bisher beschriebene Elektrodenaufbau ebenfalls flexibel und biegsam ist. Damit ist gewährleistet, dass eine derartige Elektrode Bewegungen des Körpers bzw. der unter der Haut liegenden Muskeln problemlos nachvollziehen kann, ohne dass es zu einer Ablösung oder Verschiebung der Elektrode relativ zum Körper des Trägers der Elektrode kommt. Damit ist gewährleistet, dass unabhängig von der Art und Stärke der Bewegungen des Trägers eine optimale Kontaktierung gewährleistet ist, so dass die Therapie bzw. Überwachung durch die durch die Elektrode auf den Körper übertragenen oder durch die Elektrode vom Körper aufgenommenen Signale in optimaler Form stattfinden kann.

Auch die Elektrodenkontaktierungsschicht ist wie das Nanofaservlies teilweise von der Polymerschicht umgeben und in diese eingebettet. Auch die Elektrodenkontaktierungsschicht ragt jedoch aus der Polymerschicht heraus und zwar auf der der Haut abgewandten Seite der Polymerschicht, also auf der dem Nanofaservlies gegenüberliegenden Seite.

Als besonders vorteilhaft hat sich herausgestellt, wenn die Elektrodenkontaktierungsschicht eine Schicht eines leitfähigen Textils umfasst. Dabei kann es sich beispielsweise um ein mit einem Metall, insbesondere mit Silber, beschichtetes Textil handeln.

Die Verwendung eines derartigen metallisch beschichteten Textils hat eine ganze Reihe von Vorteilen. Zum einen ist auch diese Schicht biegsam und flexibel, so dass der Elektrodenaufbau noch immer Bewegungen des Körpers des Patienten folgen kann, ohne dass durch die dadurch hervorgerufene mechanische Beanspruchung eine elektrische Kontaktierung beeinträchtigt würde. Zum anderen kann an einem derartigen beschichteten Textil in besonders vorteilhafter Weise ein Anschlussdraht angebracht werden.

Ein derartiges metallisch beschichtetes Textil verfügt über eine Vielzahl einzelner beschichteter Fasern, die jede für sich als Stromleitung funktionieren können. Aufgrund der sehr großen Vielzahl an Fasern und somit an Stromleitungskanälen, ist es für die Kontaktierung unerheblich, wenn einige dieser Kontakte beispielsweise aufgrund von gebrochener oder abgeplatzter metallischer Beschichtung nicht mehr elektrisch leitend sind. Wird nun auf ein derartige Textil, beispielsweise ein Gewebe, der Anschlussdraht gelegt, kommt es zwischen den verschiedenen Litzen des Anschlussdrahtes und den Fasern des beschichteten Gewebes bzw. des beschichteten Textils zu einer Vielzahl von Kontakten. Daher ist es auch hier unerheblich, wenn ein bestimmter Anteil dieser Kontakte sich löst, bricht oder aufgrund sonstiger Einflüsse nicht mehr funktioniert und den elektrischen Strom leitet. Eine Kontaktierung ist weiterhin gewährleistet, da es für die Leitung durch Elektronenfluss im Prinzip ausreicht, wenn einer der Kontakte bestehen bleibt. Auf diese Weise wird eine insbesondere gegen mechanische Beanspruchung sehr stabile Kontaktierung erreicht, ohne die Beweglichkeit, Flexibilität und Elastizität der Elektrode einzuschränken.

Die Elektrodenkontaktierungsschicht kann alternativ dazu auch eine auf eine Trägerfolie gedruckte metallische Tinte, insbesondere eine Silbertinte, umfassen. Dabei kann die Anzahl der Kontakte zwischen dem aufgedruckten Muster und einem beispielsweise aufgelegten Anschlussdraht bzw. dessen Litzen über die Wahl des Musters gesteuert werden. Wird hier beispielsweise ein filigranes und weitverzweigtes Muster verwendet, kann auch auf diese Weise eine Vielzahl von Kontakten geschaffen werden, von denen nur ein geringer Anteil zu funktionieren braucht, um eine optimale Kontaktierung der Elektrode zu gewährleisten.

In einer besonders vorteilhaften Ausführungsform enthält die elektrisch leitfähige Polymerschicht als leitfähige Partikel insbesondere leitfähige Pigmente, Leitruß, Graphit, CNTs oder dendritische metallische Partikel. CNT bezeichnet dabei Kohlenstoffnanoröhrchen (Carbon Nano Tubes). Insbesondere die Verwendung von Kombinationen von plättchenförmigen und nahezu kugelförmigen Partikeln hat sich bewährt. Auf diese Weise lässt sich besonders sicher und einfach bei einem relativ geringen Füllgrad die Perkolationsschwelle überschreiten, so dass es zu einer elektrischen Leitfähigkeit durch die gesamte Polymerschicht kommt.

Insbesondere die Verwendung leitfähiger Pigmente hat zudem den Vorteil, dass dadurch die Farbe der Elektrode frei wählbar ist. Elektroden für verschiedene Verwendungszwecke weisen unterschiedliche Eigenschaften, beispielsweise unterschiedliche elektrische Leitfähigkeit auf. So ist beispielsweise eine Elektrode zum Abnehmen elektrischer Signale von der Haut des Patienten mit einer höheren elektrischen Leitfähigkeit ausgestattet als eine Elektrode, durch die elektrische Signale auf die Haut übertragen werden sollen. Wird nun beispielsweise die Leitfähigkeit des Polymers durch die Verwendung von Graphit oder Leitruß hergestellt, ist die Farbe dadurch festgelegt. Eine optische Unterscheidung der verschiedenen Elektroden für die verschiedenen Anwendungsbereiche ist so nicht möglich. Ist die Elektrode einmal aus der Verpackung genommen, kann der Verwender nicht mehr entscheiden, wofür die Elektrode gedacht ist. Wird dann beispielsweise aus Versehen eine Elektrode zum Abnehmen elektrischer Signale als Stimulationselektrode verwendet, kann es aufgrund der erhöhten Leitfähigkeit bei gleicher angelegter Spannung zu einem zu starken Stromfluss kommen, der für den Träger unangenehm ist. Insbesondere kann es zu Schmerzen und Verbrennungen kommen.

Durch die Verwendung leitfähiger Partikel in der Polymerschicht kann die Verwendung von Ruß oder Graphit vermieden werden. Ruß oder Graphit bestehen in der Regel aus Nanopartikeln, die so klein sind, dass sie in den menschlichen Körper eindringen können. Dazu können sie beispielsweise durch Abrieb in die Luft gelangen und von dort über die Atemwege aufgenommen werden oder auf sonstige Weise in den menschlichen Körper gelangen. Die von diesen Nanopartikeln potenziell ausgehende Gefährdung wird durch die Verwendung der leitfähigen Partikel, die beispielsweise eine Größe im Mikrometerbereich aufweisen, sicher vermieden. Zudem ist es durch die Verwendung beispielsweise leitfähiger Pigmente möglich, die Elektroden auch nach ästhetischen Gesichtspunkten zu gestalten.

Durch die Verwendung von leitfähigen Pigmenten in der leitfähigen Polymerschicht ist die Farbe dieser Polymerschicht frei wählbar, so dass beispielsweise anhand der Farbe der Elektrode vom Benutzer selbst entschieden werden kann, ob es sich um eine Elektrode zum Abnehmen elektrischer Signale oder beispielsweise eine Stimulationselektrode handelt, mit der elektrische Signale auf die Haut des Patienten übertragen werden sollen. Damit werden Fehlverwendungen verhindert.

Ein erfindungsgemäßes Verfahren zum Herstellen einer derartigen Elektrode umfasst die folgenden Schritte:
a) Gießen oder Rakeln zumindest eines ersten Teils einer leitfähigen Polymerschicht aus einem Polymer, das leitfähige Partikel enthält,
b) Eindrücken einer ersten Schicht, beispielsweise eines Faservlieses, insbesondere eines Nanofaservlieses in die Polymerschicht, so dass die erste Schicht teilweise von der Polymerschicht durchdrungen ist und an einer ersten Seite aus der Polymerschicht herausragt.

Das Polymer wird im flüssigen bearbeitbaren Zustand auf eine Arbeitsfläche gegossen. Optional kann zusätzlich oder alternativ dazu auch durch Rakeln eine homogone Schicht erzeugt werden, in der die leitfähigen Partikel, insbesondere Pigmente, in einer Vorzugsorientierung ausgerichtet werden. Solange es in diesem Zustand ist, wird nun das Nanofaservlies auf die Polymerschicht aufgelegt und eingedrückt. Gegebenenfalls wird dazwischen etwas gewartet, so dass die Polymerschicht etwas anhärten kann. Alternativ dazu kann die Viskosität der Polymerschicht auch so gewählt werden, dass die erste Schicht, beispielsweise das Nanofaservlies, sofort eingedrückt werden kann. Auf diese Weise kann sehr genau eingestellt werden, wie groß die Eindringtiefe der ersten Schicht in der Polymerschicht ist.

Vorzugsweise umfasst das Verfahren folgende weiteren Schritte:
c) Anhärtenlassen des zumindest ersten Teils der leitfähigen Polymerschicht,
d) Gießen oder Rakeln eines zweiten Teils der leitfähigen Polymerschicht auf eine der ersten Seite gegenüberliegende zweite Seite der Polymerschicht und
e) Eindrücken einer Elektrodenkontaktierungsschicht in den zweiten Teil der Polymerschicht, so dass die Elektrodenkontaktierungsschicht teilweise in der Polymerschicht eingebettet ist und an der zweiten Seite aus der Polymerschicht herausragt.

Nachdem folglich die erste Schicht, beispielsweise das Nanofaservlies, in den ersten Teil des leitfähigen Polymers eingedrückt wurde, lässt man das Polymer soweit anhärten, dass man die beiden Schichten, also das Nanofaservlies und die leitfähige Polymerschicht gemeinsam bewegen und insbesondere umdrehen kann. Nach dem Umdrehen kann auf die jetzt oben liegende ursprüngliche Unterseite des ersten Teils der leitfähigen Polymerschicht ein zweiter Teil der leitfähigen Polymerschicht aufgebracht werden. Auch dieser wird in flüssigem Zustand aufgebracht, so dass eine Elektrodenkontaktierungsschicht, die beispielsweise in Form eines leitfähigen Textils vorliegen kann, in diesen zweiten Teil eingedrückt werden kann. Auch hier kann sehr genau eingestellt werden, wie tief die Elektrodenkontaktierungsschicht in das Polymer eindringen soll und wie weit sie aus dem Polymer herausragt. Anschließend kann auf die Elektrodenkontaktierungsschicht, beispielsweise das metallisch beschichtete leitfähige Textil ein Anschlussdraht, insbesondere mit mehreren Litzen, angebracht werden, um so eine optimale Kontaktierung an möglichst vielen Stellen zu gewährleisten.

Abschließend kann auf die Seite der Elektrode, die im Verwendungsfall der Haut abgewandt ist, also auf die Seite, an der die Elektrodenkontaktierungsschicht aus der Polymerschicht herausragt, beispielsweise ein selbstklebendes, elektrisch isolierendes Textil geklebt werden. Damit werden ungewollte Kontakte, beispielsweise zur Kleidung des Patienten verhindert.

Die für die leitfähige Polymerschicht verwendbaren leitfähigen Partikel umfassen insbesondere dendritische metallische Partikel, bevorzugt Silber, Leitruß, Graphit, CNTs oder leitfähige Pigmente. Diese können beispielsweise aus einem Glimmerplättchen und einem Glimmerkügelchen als Kern bestehen, die beispielsweise mit einer mit Antimon dotierten Zinnoxidschicht beschichtet sind. Insbesondere die Kombination von Plättchen und Kügelchen als zugesetzte Partikel hat sich bewährt, um die Perkolationsschwelle einfach und sicher überschreiten zu können.

Mit einer erfindungsgemäßen Elektrode werden somit alle Nachteile aus dem Stand der Technik überwunden. Es kommt nicht zu einem Ankleben der Elektrode an der Haut des Patienten, so dass das unangenehme Lösen und Ankleben, das durch Bewegung hervorgerufen werden kann, gar nicht auftritt. Zudem ist durch den flexiblen und elastischen Aufbau der Elektrode gewährleistet, dass selbst bei starker mechanischer Beanspruchung die elektrische Kontaktierung gewährleistet bleibt. Dies gilt insbesondere auch für die Ausgestaltungen, in denen ein Anschlussdraht beispielsweise auf einem metallisch leitenden Textil angebracht wird. Durch die Vielzahl der verschiedenen Kontaktstellen ist die Verbindung unanfällig gegen mechanische Belastungen.

Vorteilhafterweise wird der zumindest erste Teil der leitfähigen Polymerschicht auf eine Elektrodenkontaktierungsschicht gegossen. Dadurch ist es möglich, eine Elektrode gemäß einem Ausführungsbeispiel der vorliegenden Erfindung einfach, schnell und kostengünstig herzustellen. Es wird insbesondere das zweimalige Aufgießen und/oder Rakeln eines Teils der leitfähigen Polymerschicht verhindert, da die gesamte Polymerschicht in einem Durchgang aufgebracht werden kann.

Über die Viskosität der aufgebrachten Polymerschicht kann bestimmt werden, wie weit die Elektrodenkontaktierungsschicht von der aufgebrachten Polymerschicht durchdrungen werden soll. Gleichzeitig kann auch bestimmt werden, wie weit die später aufgebrachte erste Schicht in die bereits aufgebrachte flüssige Polymerschicht eindringen soll. Die Viskosität der Polymerschicht kann beispielsweise über den Anteil von Lösungsmitteln innerhalb der Schicht oder über Zusätze bestimmt werden. Zudem kann die Eindringtiefe über den Anpressdruck, mit dem eine Schicht auf die darunterliegende Schicht aufgebracht wird, geregelt werden.

Insbesondere, wenn als erste Schicht ein Faservlies, beispielsweise ein Nanofaservlies verwendet wird, ist es möglich, dieses aus einem Polymer herzustellen, das auch dem Grundpolymer der leitfähigen Polymerschicht entspricht. In diesem Fall ist es besonders einfach möglich, eine Verbindung zwischen dem als erste Schicht fungierende Nanofaservlies und der Polymerschicht aus leitfähigem Polymer herzustellen. Dabei wird beispielsweise die Polymerschicht auf eine Arbeitsplatte aufgebracht. Sie wird leicht angehärtet, bis der Anteil der noch in der Polymerschicht vorhandenen Lösungsmittel einen vorbestimmten Wert aufweist. Anschließend wird das Nanofaservlies, das aus dem gleichen Polymer besteht, auf die Polymerschicht aufgebracht. Durch die in der Polymerschicht noch enthaltenen Lösungsmittel wird auch das Faservlies leicht angelöst, so dass es sich mit der noch ebenfalls angelösten Polymerschicht verbinden kann. Man spricht in diesem Fall vom Kaltschweißen. Auf diese Weise wird eine besonders gute Verbindung zwischen den einzelnen Schichten erreicht.

Wie bereits dargelegt, werden insbesondere Nanofaservliese durch Elektrospinning hergestellt. Da es sich bei der leitfähigen Polymerschicht um eine elektrisch leitfähige Schicht handelt, ist es möglich, das Nanofaservlies direkt auf diese elektrisch leitfähige Schicht aufzuspinnen. Auch dadurch werden Arbeitsschritte eingespart, so dass das Verfahren insgesamt beschleunigt und somit die Herstellungskosten gesenkt werden.

Wird hierfür das noch nicht vollständig ausgehärtete Polymer der aufgebrachten Polymerschicht verwendet, kommt es gleichzeitig zu der beschriebenen Kaltschweißverbindung, so dass neben der einfachen, schnellen und kostengünstigen Herstellung auch noch eine besonders gute Verbindung zwischen den einzelnen Schichten erreicht wird.

Eine Elektrode gemäß einem Ausführungsbeispiel der vorliegenden Erfindung kann insbesondere in einer Bandage verwendet werden. Eine erfindungsgemä Bandage umfasst daher mindestens eine der beschriebenen Elektroden. Durch die vorliegende Erfindung können Elektroden so ausgebildet werden, dass sie von selbst nicht an der Haut des Patienten haften. Daher besteht über eine Bandage eine gute Möglichkeit, die Position der Elektroden am Körper des Patienten festzulegen, ohne die Haut zu reizen oder zu Hautirritationen zu führen. Als besonders vorteilhaft hat sich dabei herausgestellt, wenn die wenigstens eine Elektrode lösbar, beispielsweise mittels eines Klettverschlusses, an einem Gurt der Bandage angeordnet ist. Auf diese Weise ist es möglich, die Elektroden nach Gebrauch, beispielsweise nach Abschluss der Therapie, von der Bandage zu lösen und an einer anderen Bandage gegebenenfalls für völlig andere Körperteile weiter zu verwenden. Daher sind derartige Elektroden über einen langen Zeitraum gegebenenfalls auch an verschiedenen Körperteilen für verschiedene Therapien bei gegebenenfalls sogar verschiedenen Patienten einsetzbar.

Mit Hilfe einer Zeichnung wird nachfolgend ein Ausführungsbeispiel der vorliegenden Erfindung näher erläutert. Es zeigt:
Figur 1 - eine schematische Darstellung einer Elektrode gemäß einem ersten Ausführungsbeispiel der vorliegenden Erfindung; und
Figur 2 - eine Elektrode gemäß einem Ausführungsbeispiel der vorliegenden Erfindung in einer Explosionsdarstellung.

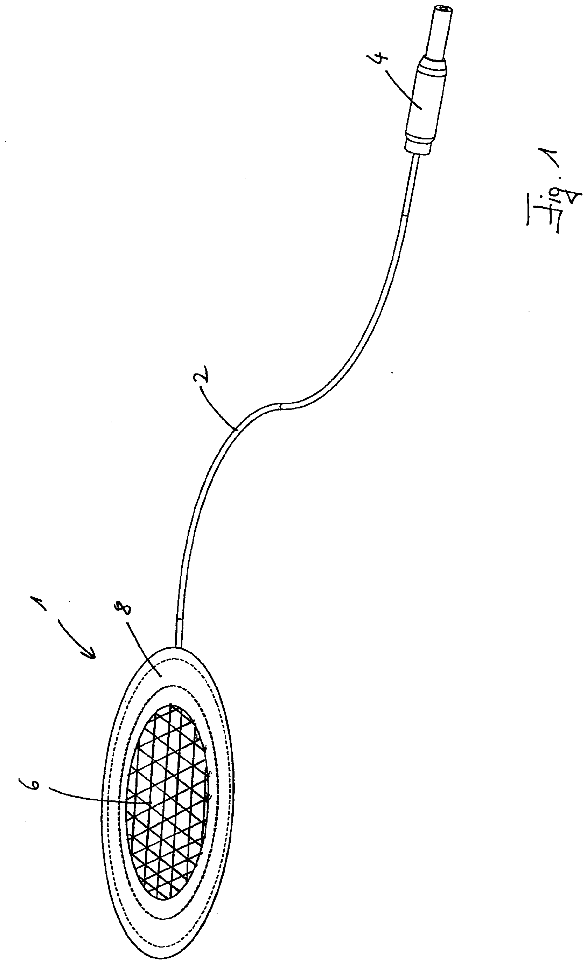

Figur 1 zeigt eine Elektrode 1 gemäß einem ersten Ausführungsbeispiel der vorliegenden Erfindung. Man erkennt einen Anschlussdraht 2, an dessen einem Ende ein Stecker 4 angeordnet ist. Über diesen kann er beispielsweise mit einer elektrischen Steuerung oder einem weiteren Gerät verbunden werden, das von der Elektrode 1 abgenommene elektrische Signale weiterverarbeitet oder durch den Anschlussdraht 2 elektrische Impulse zur Elektrode 1 sendet, um diese an den Körper eines Patienten weiterzugeben. Anstelle der in Figur 1 gezeigten Ausführungsform ist es natürlich auch möglich, andere Formen der elektrischen Kontaktierung, die insbesondere kompatibel mit bestehenden Geräten, beispielsweise EKG, EEG oder EMG sind, vorzusehen.

An dem dem Stecker 4 abgewandten Ende des Anschlussdrahtes 2 befindet sich die Elektrode 1. Im mittleren Bereich der Elektrode 1 erkennt man ein Nanofaservlies 6, das im Verwendungsfall der Elektrode 1 mit der Haut eines Patienten in Kontakt kommt. Um diesen Kontaktbereich herum befindet sich eine Folie 8, die beispielsweise aus einem Polyurethan bestehen kann. Diese ist im gezeigten Ausführungsbeispiel dafür vorgesehen, die Fläche der Elektrode, über die elektrische Signale an die Haut abgegeben werden können, zu begrenzen und gleichzeitig zu verhindern, dass eine unterhalb des Nanofaservlieses 6 befindliche leitfähige Polymerschicht 10 mit der Haut des Patienten in Kontakt kommt.

Die geometrische Form der Elektrode ist völlig frei wählbar und kann an die gewünschten Stromstärken bzw. Spannungen, die Körperteile an denen die Elektrode verwendet werden soll, und sonstige individuelle Umstände angepasst werden.

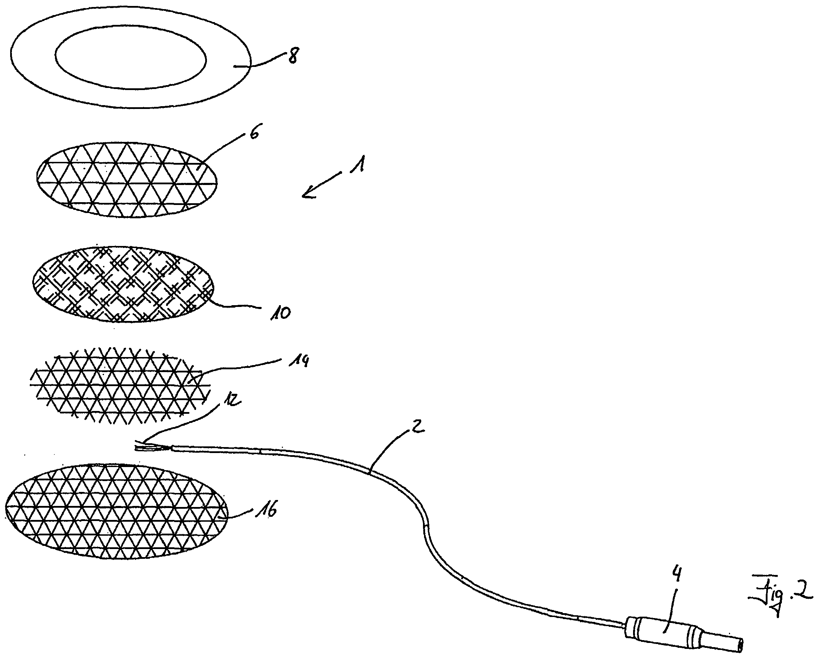

Figur 2 zeigt die in Figur 1 dargestellte Elektrode in einer Explosionsdarstellung. Man erkennt wieder einen Anschlussdraht 2, an dessen einem Ende sich der Stecker 4 befindet. Das andere Ende ist in Figur 2 noch nicht mit der Elektrode 1 verbunden. Man sieht die einzelnen Litzen 12, die aus dem Anschlussdraht 2 herausragen. Die eigentliche Elektrode 1 ist aus fünf verschiedenen Schichten bestehend dargestellt. In der Mitte befindet sich die Polymerschicht 10, die leitfähige Partikel umfasst. Dabei ist wichtig, dass die Partikel in Form und Anzahl so gewählt werden, dass die Perkolationsschwelle überschritten wird, so dass die Leitung des elektrischen Stroms durch die gesamte Polymerschicht 10 möglich ist.

Oberhalb der Polymerschicht 10 befindet sich das Nanofaservlies 6. Im fertigen Zustand der Elektrode 1 befindet sich dieses teilweise innerhalb der Polymerschicht 10, so dass es zu einer optimalen Kontaktierung zwischen den beiden Schichten kommt. Das Nanofaservlies 6 ist dabei mit einer Ionen beinhaltenden Flüssigkeit getränkt. Es handelt sich folglich um ein ionisch leitfähiges Material, durch das elektrische Signale besonders gut auf die Haut eines Patienten übertragen werden können. Oberhalb des Nanofaservlieses ist in Figur 2, wie bereits in Figur 1, die Folie 8 zu erkennen, die beispielsweise aus einem Polyurethan bestehen kann. Damit wird zum einen wieder die Fläche der Elektrode, über die elektrische Impulse an die Haut abgegeben werden können, begrenzt und zum anderen ein direkter Kontakt zwischen der Polymerschicht 10 und der Haut verhindert. Bei der Polymerschicht 10 mit den darin enthaltenen leitfähigen Partikeln handelt es sich um einen Elektronenleiter. Der Stromfluss in dieser Schicht wird folglich durch den Transport von Elektronen und nicht, wie in dem Nanofaservlies 6, durch den Transport von Ionen erreicht. Ein direkter Kontakt zwischen der Polymerschicht 10 und der Haut des Patienten kann somit für den Patienten unangenehm und schmerzhaft sein. Kann ein derartiger Kontakt auf andere Weise sicher verhindert werden, ist die Folie 8 dabei überflüssig.

Unterhalb der Polymerschicht 10 ist in Figur 2 eine Elektrodenkontaktierungsschicht 14 gezeigt, die beispielsweise in Form eines leitfähigen Textils vorliegen kann. Dabei wird das Textil beispielsweise mit Metall bedampft oder beschichtet, so dass es elektrisch leitfähig wird. Ein derartiges Textil, das beispielsweise in Form eines beschichteten Gewebes vorliegenden kann, verfügt über eine Vielzahl möglicher elektrischer Leitungen und Kanäle. Durch das Auflegen der Litzen 12 des Anschlussdrahtes 2 auf diese Elektrodenkontaktierungsschicht werden eine Vielzahl von Kontakten gebildet, die alle in der Lage sind, den elektrischen Strom aus dem Anschlussdraht in die Elektrodenkontaktierungsschicht 14 zu leiten. Fallen nun aufgrund mechanischer Beanspruchung oder durch Herstellungsfehler einige dieser Kontakte aus, hat dies nicht einen Abriss der Kontaktierung zur Folge. Somit ist weiterhin gewährleistet, dass die Elektrode 1 funktioniert und elektrische Signale beispielsweise an den Körper des Trägers abgegeben werden können.

Den Abschluss bildet in Figur 2 eine Schicht eines elektrisch isolierenden Textils 16. Damit wird verhindert, dass zwischen der Elektrodenkontaktierungsschicht 14 und beispielsweise der Kleidung des Patienten elektrische Kontakte entstehen, die störenden Einfluss auf die zu übertragenden elektrischen Signale haben können.

Bei der Herstellung einer derartigen Elektrode 1 wird zunächst ein Teil der Polymerschicht 10 in flüssigem Zustand gegossen und gegebenenfalls zu einer Fläche gerakelt. In dieses noch flüssige Polymer der Polymerschicht 10 mit den darin bereits enthaltenen leitfähigen Partikeln wird die erste Schicht, beispielsweise in Form des Nanofaservlieses 6, das beispielsweise in Form eines Elektrospinvlieses vorliegen kann, eingedrückt. Über den Druck und/oder die Viskosität der Polymerschicht, die beispielsweise über den Lösungsmittelgehalt gesteuert werden kann, kann sehr genau bestimmt werden, die tief das Nanofaservlies 6 in die Polymerschicht 10 eindringt, so dass auch genauso genau festgelegt werden kann, wie weit es aus dem Polymer herausragt. Damit ist eine optimale Kontaktierung möglich. In einem weiteren Schritt wird das Polymer soweit ausgehärtet, dass es mit dem darin befindlichen Nanofaservlies 6 bewegbar ist. Insbesondere kann es nun umgedreht werden, so dass das Nanofaservlies 6 unten unterhalb des ersten Teils der Polymerschicht 10 zu liegen kommt. Auf den bereits leicht ausgehärteten ersten Teil der Polymerschicht 10 wird nun ein zweiter Teil der Polymerschicht 10 gegossen, in den ebenfalls im flüssigen Zustand die Elektrodenkontaktierungsschicht 14, beispielsweise in Form eines metallisch beschichteten Textils geblickt wird. Auch diese Schicht wird in das flüssige Polymer der Polymerschicht 10 eingedrückt, so dass in optimaler Weise festgelegt werden kann, wie tief die Schicht in das Polymer eindringt und wie weit sie aus diesem heraussteht.

Nachdem in einem weiteren Verfahrensschritt die beiden Polymerschichen 10 soweit ausgehärtet sind, dass die Elektrode 1 weiterverarbeitet werden kann, kann auf die nun mit der Polymerschicht 10 verbundene Elektrodenkontaktierungsschicht 14, und nach Zuschnitt der Elektrode, der Anschlussdraht 2 mit seinen Litzen 12 gelegt werden. Dabei kommt es zu den bereits genannten vielen elektrischen Kontakten, so dass der Ausfall einiger dieser Kontakte nicht den vollständigen Ausfall der Elektrode zur Folge hat.

### Bezugszeichenliste

- 1 -: Elektrode
- 2 -: Anschlussdraht
- 4 -: Stecker
- 6 -: Nanofaservlies
- 8 -: Folie
- 10 -: Polymerschicht
- 12 -: Litze
- 14 -: Elektrodenkontaktierungsschicht
- 16 -: elektrisch isolierendes Textil

## Patentansprüche

1. Elektrode (1) zum transkutanen Übertragen elektrischer Signale mit
a) einer ersten Schicht und
b) einer elektrisch leitfähigen Polymerschicht (10), die leitfähige Partikel enthält,
wobei die erste Schicht teilweise von der Polymerschicht (10) durchdrungen ist, so dass sie aus der Polymerschicht (10) an zumindest einer ersten Seite herausragt, **dadurch gekennzeichnet, dass** die erste Schicht ausgebildet ist, eine Flüssigkeit zumindest auch aufgrund einer Kapillarkraft festzuhalten, und dass die Elektrode (1) eine Elektrodenkontaktierungsschicht (14) aufweist, die teilweise in die Polymerschicht (10) eingebettet ist, so dass sie auf einer der ersten Seite gegenüberliegenden zweiten Seite aus der Polymerschicht (10) herausragt, wobei es sich bei der ersten Schicht um ein Faservlies oder einen Schwamm handelt.

2. Elektrode (1) nach Anspruch 1, **dadurch gekennzeichnet, dass** es sich bei der ersten Schicht um ein Nanofaservlies (6) handelt.

3. Elektrode (1) nach Anspruch 2, **dadurch gekennzeichnet, dass** an der Elektrodenkontaktierungsschicht (14) ein Anschlussdraht (2) angeordnet ist.

4. Elektrode (1) nach einem der vorstehenden Ansprüche, **dadurch gekennzeichnet, dass** die Elektrodenkontaktierungsschicht (14) eine Schicht eines leitfähigen Textils umfasst.

5. Elektrode (1) nach Anspruch 4, **dadurch gekennzeichnet, dass** das leitfähige Textil ein mit einem Metall, insbesondere Silber, beschichtetes Textil ist.

6. Elektrode (1) nach einem der vorstehenden Ansprüche, **dadurch gekennzeichnet, dass** die Elektrodenkontaktierungsschicht (14) eine auf einer Trägerfolie gedruckte metallische Tinte, insbesondere eine Silbertinte, umfasst.

7. Elektrode (1) nach einem der vorstehenden Ansprüche, **dadurch gekennzeichnet, dass** die elektrisch leitfähige Polymerschicht (10) als leitfähige Partikel leitfähige Pigmente, Graphit, Leitruß, CNTs o-der dendritische metallische Partikel enthält.

8. Verfahren zum Herstellen einer Elektrode (1) nach einem der vorstehenden Ansprüche, das die folgenden Schritte umfasst:
a) Gießen oder Rakeln zumindest eines ersten Teils einer leitfähigen Polymerschicht (10) aus einem Polymer, das leitfähige Partikel enthält,
b) Eindrücken einer ersten Schicht, beispielsweise eines Faservlieses, insbesondere eines Nanofaservlieses (6) in die Polymerschicht (10), so dass die erste Schicht teilweise von der Polymerschicht (10) durchdrungen ist und an einer ersten Seite aus der Polymerschicht (10) herausragt.

9. Verfahren nach Anspruch 8, **gekennzeichnet durch** die weiteren Schritte:
c) Anhärtenlassen des ersten Teils der leitfähigen Polymerschicht (10),
d) Gießen oder Rakeln eines zweiten Teils der leitfähigen Polymerschicht (10) auf einer der ersten Seite gegenüberliegende zweite Seite der Polymerschicht (10) und
e) Eindrücken einer Elektrodenkontaktierungsschicht (14) in den zweiten Teil der Polymerschicht (10), so dass die Elektrodenkontaktierungsschicht (14) teilweise in der Polymerschicht (10) eingebettet ist und an der zweiten Seite aus der Polymerschicht (10) herausragt.

10. Verfahren nach Anspruch 8, **dadurch gekennzeichnet, dass** der zumindest erste Teil der leitfähigen Polymerschicht (10) auf eine Elektrodenkontaktierungsschicht (14) gegossen oder gerakelt wird.

11. Bandage mit wenigstens einer Elektrode nach einem der Ansprüche 1 bis 7.

12. Bandage nach Anspruch 11, **dadurch gekennzeichnet, dass** die wenigstens eine Elektrode lösbar, beispielsweise mittels eines Klettverschlusses, an einem Gurt der Bandage angeordnet ist.

## Claims

1. An electrode (1) for transcutaneously transmitting electronic signals, comprising:
a) a first layer and
b) an electrically conductive polymer (10) layer containing conductive particles,
the first layer being partially permeated by the polymer layer (10) such that it protrudes out of said polymer layer (10) on at least one first side, **characterized in that** the first layer is designed to retain a liquid, at least also as a result of a capillary force, and that the electrode (1) comprises an electrode contacting layer (14) which is partially embedded in the polymer layer (10), such that it protrudes out of the polymer layer (10) on a second side opposite the first side, wherein the first layer is a nonwoven or a sponge.

2. The electrode (1) according to claim 1, **characterized in that** the first layer is a nano nonwoven (6).

3. The electrode (1) according to claim 2, **characterized in that** a connecting wire (2) is arranged on the electrode contacting layer (14).

4. The electrode (1) according to one of the preceding claims, **characterized in that** the electrode contacting layer (14) comprises a layer of conductive textile.

5. The electrode (1) according claim 4, **characterized in that** the conductive textile is a textile that is coated with a metal, in particular silver.

6. The electrode (1) according to one of the preceding claims, **characterized in that** the electrode contacting layer (14) comprises a metallic ink, in particular a silver ink, printed onto a carrier film.

7. The electrode (1) according to one of the preceding claims, **characterized in that** the electrically conductive polymer layer (10) contains conductive pigments, graphite, electrically conductive carbon black, CNTs or dendritic metallic particles that act as conductive particles.

8. A method for producing an electrode (1) according to one of the preceding claims, comprising:
a) Moulding or spreading at least a first part of a conductive polymer layer (10) made from a polymer that contains conductive particles,
b) Pushing a first layer, such as for example nonwoven, in particular a nano nonwoven (6) into the polymer layer (10), such that the first layer is partially permeated by the polymer layer (10) and protrudes out of the polymer layer (10) on a first side.

9. The method according to claim 8, further comprising:
c) Allowing the first part of the conductive polymer layer (10) to harden,
d) Moulding or spreading a second part of the conductive polymer layer (10) on a second side of the polymer layer (10) that is opposite the first side, and
e) Pushing an electrode contacting layer (14) into the second part of the polymer layer (10), such that the electrode contacting layer (14) is partially embedded in the polymer layer (10) and protrudes out of the polymer layer (10) on the second side.

10. The method according to claim 8, **characterized in that** the at least first part of the conductive polymer layer (10) is moulded or spread on an electrode contacting layer (14).

11. A bandage with at least one electrode according to claims 1 to 7.

12. The bandage according to claim 11, **characterized in that** the at least one electrode is arranged on one strap of the bandage such that the at least one electrode can be detached, for example by a hook-and-loop fastener.

## Revendications

1. Electrode (1) pour la transmission transcutanée de signaux électriques, com portant
a) une première couche et
b) une couche de polymère (10) électriquement conductrice, qui contient des particules conductrices,
la première couche étant partiellement traversée par la couche de polymère (10), de sorte qu'elle dépasse de la couche de polymère (10) sur au moins une première face,
**caractérisée en ce que** la première couche est réalisée pour retenir un liquide au moins également en raison d'une force capillaire, et **en ce que** l'électrode (1) présente une couche de mise en contact d'électrode (14) qui est partiellement noyée dans la couche de polymère (10), de sorte qu'elle dépasse de la couche de polymère (10) sur une deuxième face opposée à la première face, la première couche étant une nappe de fibres ou une éponge.

2. Electrode (1) selon la revendication 1,
**caractérisée en ce que** la première couche est une nappe de nanofibres (6).

3. Electrode (1) selon la revendication 2,
**caractérisée en ce qu'**un fil de connexion (2) est disposé sur la couche de mise en contact d'électrode (14).

4. Electrode (1) selon l'une des revendications précédentes,
**caractérisée en ce que** la couche de mise en contact d'électrode (14) comprend une couche d'un textile conducteur.

5. Electrode (1) selon la revendication 4,
**caractérisée en ce que** le textile conducteur est un textile revêtu d'un métal, en particulier d'argent.

6. Electrode (1) selon l'une des revendications précédentes,
**caractérisée en ce que** la couche de mise en contact d'électrode (14) comprend une encre métallique, en particulier une encre à base d'argent, imprimée sur un film support.

7. Electrode (1) selon l'une des revendications précédentes,
**caractérisée en ce que** la couche de polymère (10) électriquement conductrice contient comme particules conductrices des pigments conducteurs, du graphite, du noir de carbone conducteur, des CNT ou des particules métalliques dendritiques.

8. Procédé de fabrication d'une électrode (1) selon l'une des revendications précédentes, comprenant les étapes suivantes consistant à :
a) couler ou racler au moins une première partie d'une couche de polymère conductrice (10) en un polymère contenant des particules conductrices,
b) enfoncer une première couche, par exemple une nappe de fibres, en particulier une nappe de nanofibres (6), dans la couche de polymère (10), de sorte que la première couche est partiellement traversée par la couche de polymère (10) et dépasse de la couche de polymère (10) sur une première face.

9. Procédé selon la revendication 8,
**caractérisé par** les autres étapes consistant à :
c) laisser durcir la première partie de la couche de polymère conductrice (10),
d) couler ou racler une deuxième partie de la couche de polymère conductrice (10) sur une deuxième face de la couche de polymère (10) opposée à la première face, et
e) enfoncer une couche de mise en contact d'électrode (14) dans la deuxième partie de la couche de polymère (10), de sorte que la couche de mise en contact d'électrode (14) est partiellement noyée dans la couche de polymère (10) et dépasse de la couche de polymère (10) sur la deuxième face.

10. Procédé selon la revendication 8,
**caractérisé en ce que** ladite au moins première partie de la couche de polymère conductrice (10) est coulée ou raclée sur une couche de mise en contact d'électrode (14).

11. Bandage comportant au moins une électrode selon l'une des revendications 1 à 7.

12. Bandage selon la revendication 11,
**caractérisé en ce que** ladite au moins une électrode est disposée de manière amovible, par exemple au moyen d'une fermeture velcro, sur une sangle du bandage.
